# EUROPÄISCHE PATENTSCHRIFT

(11) **EP 0 554 786 B1**
(45) Veröffentlichungstag und Bekanntmachung des Hinweises auf die Patenterteilung: **29.11.1995**
(21) Anmeldenummer: 93101250.4
(22) Anmeldetag: 28.01.1993
(51) Int. Cl.: C07C 255/46, C07C 253/10

(54) **Verfahren zur kontinuierlichen Herstellung von 3-Cyano-3,5,5-trimethylcyclohexanon**
Process for the continuous preparation of 3-cyano-3,5,5-trimethyl-cyclohexanone
Procédé pour la préparation continue de la 3-cyano-3,5,5-triméthyl-cyclohexanone

(30) Priorität: 07.02.1992 DE 4203456
(43) Veröffentlichungstag der Anmeldung: 11.08.1993
(73) Patentinhaber: BASF Aktiengesellschaft, 67063 Ludwigshafen (DE)
(72) Erfinder: Pander, Hans Joachim, W-6701 Roedersheim-Gronau (DE); Siegel, Hardo, Dr., W-6720 Speyer (DE); Woerz, Otto, Dr., W-6701 Friedelsheim (DE)
(74) Vertreter: Isenbruck, Günter, Dr.

(56) Entgegenhaltungen:
- EP-A- 0 028 179
- DE-B- 1 240 854
- US-A- 5 011 968

## Beschreibung

Die Erfindung betrifft ein Verfahren zur kontinuierlichen Herstellung von 3-Cyano-3,5,5-trimethylcyclohexanon durch basenkatalysierte Umsetzung von Isophoron und Cyanwasserstoff in zwei getrennten Reaktionszonen.

Es ist bekannt, 3-Cyano-3,5,5-trimethylcyclohexanon durch Umsetzung von Isophoron mit Cyanwasserstoff in Gegenwart stark alkalischer Katalysatoren herzustellen. Aus der DE-A-10 85 871 ist ein Verfahren bekannt, bei dem α, β-ungesättigte Ketone und Cyanwasserstoff in Gegenwart eines stark alkalischen Katalysators und eines polaren Lösungsmittels zu den entsprechenden Cyanoketonen umgesetzt werden. Die Umsetzung wird vorzugsweise diskontinuierlich durchgeführt. Bei der beispielhaft angeführten Umsetzung von Isophoron und Cyanwasserstoff in Gegenwart von 1,15 % Kaliumcarbonat wird in Dimethylacetamid als Lösungsmittel 3-Cyano-3,5,5-trimethyl-cyclohexanon in einer Ausbeute von 70 % erhalten.

Aus der DE-A-12 40 854 ist ein Verfahren bekannt, bei dem in Abwesenheit eines Lösungsmittels bei Katalysatorkonzentrationen von 10⁻¹ bis 10⁻³ Gew-% bezogen auf das Reaktionsgemisch Ausbeuten an 3-Cyano-3,5,5-trimethylcyclohexanon von 96 % erreicht werden. Beispielhaft ist auch ein kontinuierliches Verfahren zur Herstellung von 3-Cyano-3,5,5-trimethyl-cyclohexanon aus Isophoron und Cyanwasserstoff in drei hintereinander geschalteten Rührkesseln beschrieben. Die mittlere Verweilzeit beträgt 4 Stunden im ersten Reaktor und je eine Stunde in den beiden Nachreaktoren.

In der DE-A-12 40 521 wird ein Verfahren beschrieben, bei dem die Umsetzung von Isophoron oder Mesityloxid mit Cyanwasserstoff in Gegenwart von alkalischen Katalysatoren erfolgt, die auf festen Tägermaterialien aufgebracht sind. Um zu guten Ausbeuten an 3-Cyano-3,5,5-trimethylcyclohexanon zu zu gelangen, muß der HCN-Anteil im Reaktionsgemisch niedrig gehalten werden.

Der HCN-Umsatz liegt beispielhaft zwischen 94 und 98 %, das entspricht HCN-Konzentrationen im ausreagierten Reaktionsgemisch von 0,1 bis 0,4 %. Diese relativ hohen HCN-Konzentrationen erfordern im Falle einer technischen Nutzung des Verfahrens einen separaten Reinigungsschritt.

Der vorliegenden Erfindung lag daher die Aufgabe zugrunde, den zuvor genannten Nachteilen abzuhelfen.

Demgemäß wurde ein neues und verbessertes Verfahren zur kontinuierlichen Herstellung von 3-Cyano-3,5,5-trimethylcyclohexanon durch basenkatalysierte Umsetzung von Isophoron und Cyanwasserstoff gefunden, welches dadurch gekennzeichnet ist, daß man in zwei getrennten Reaktionszonen
a) mit im wesentlichen vollständiger Rückvermischung und anschließend
b) im wesentlichen ohne Rückvermischung
arbeitet.

Das erfindungsgemäße Verfahren läßt sich wie folgt durchführen:
Die kontinuierliche basenkatalysierte Umsetzung von Isophoron und Cyanwasserstoff zu 3-Cyano-3,5,5-trimethylcyclohexanon in zwei getrennten Reaktionszonen a) und b) kann im Temperaturbereich von 60 bis 250°C, bevorzugt 100 bis 210°C, besonders bevorzugt 130 bis 180°C und Drücken von 0,01 bis 5 bar, bevorzugt 0,1 bis 2 bar, besonders bevorzugt bei Normaldruck (Atmosphärendruck) durchgeführt werden. Vorteilhaft arbeitet man im Molverhältnis von Isophoron zu Cyanwasserstoff von 1,1 : 1 bis 6 : 1, vorzugsweise 1,3 : 1 bis 2,5 : 1. Die Reaktion kann in Gegenwart eines inerten Lösungsmittels erfolgen, vorzugsweise wird jedoch in Abwesenheit eines inerten Lösungsmittels gearbeitet.
a) Als Reaktor mit im wesentlichen vollständiger Rückvermischung kann z.B. ein Rührkessel, ein Mischkreis oder ein Schlaufenreaktor eingesetzt werden. Die frei werdende Reaktionswärme wird über geeignete Wärmetauscher abgeführt.
b) Als Nachreaktor eignen sich zylindrische Reaktoren mit Füllkörpern oder fest angeordneten Einbauten, die eine Rückvermischung vollständig oder teilweise verhindern. Bei der Durchführung der Synthese im Labormaßstab kann jedoch auch ein Rohrreaktor eingesetzt werden, der im turbulenten Strömungsbereich betrieben wird.

Als basische Katalysatoren sind alle Substanzen geeignet, die unter den Reaktionsbedingungen in Gegenwart von Cyanwasserstoff Cyanidionen bilden. Dazu gehören z. B. Hydroxide, Cyanide und Alkoholate der Alkali- und Erdalkalimetalle sowie quarternäre Ammoniumverbindungen. Vorzugsweise werden C₁- bis C₄-Alkalialkoholate wie Natriummethylat, Natriumethylat, Kaliummethylat, Kalium-tert.-Butylat, Lithiummethylat, besonders bevorzugt wird Natriummethylat verwendet.

Die Katalysatorkonzentration liegt zwischen 0,01 und 3 Gew.-% bezogen auf das Reaktionsgemisch. Vorzugsweise wird die Katalysatorkonzentration so gewählt, daß die von der Reaktionstemperatur und der Zusammensetzung des Reaktionsgemisches abhängige Löslichkeit des basischen Katalysators nicht überschrittten wird, das sind vorzugsweise Konzentrationen zwischen 0,01 und 0,3 Gew.-% bezogen auf das Reaktionsgemisch.

Als inerte Lösungsmittel eignen sich z.B. Dimethylformamid, Dimethylacetamid, N-Methylpyrrolidon, bevorzugt wird die Reaktion ohne Lösungsmittelzusatz durchgeführt.

Die für vollständigen HCN-Umsatz notwendige Verweilzeit ist abhängig von der Reaktionstemperatur und der Katalysatorkonzentration. Sie beträgt für den Rührreaktor in aller Regel 1 bis 4 Stunden, für den ohne Rückvermischung betriebenen Nachreaktor in aller Regel 0,2 bis 1,5 Stunden.

Das so erhaltene Reaktionsgemisch kann mit Wasser extrahiert werden, um den gelösten Katalysator zu entfernen. Der basische Katalysator kann jedoch auch durch Zusatz einer equivalenten oder überschüssigen Menge einer organischen oder anorganischen Säure neutralisiert werden. Das gewaschene oder durch Zusatz einer Säure stabilisierte Reaktionsgemisch kann anschließend durch fraktionierte Destillation gereinigt werden. Nicht umgesetztes Isophoron kann in den kontinuierlichen Reaktionsprozeß zurückgeführt.

Diese Reaktorkombination bietet bei der technischen Durchführung in Synthese gegenüber einer mehrstufigen Kühlerkesselkaskade erhebliche wirtschaftliche und sicherheitstechnische Vorteile.
Die problemlose Durchführbarkeit der Synthese von Isophoronnitril aus Isophoron und Cyanowasserstoff in dieser Reaktionskombination war nicht vorhersehbar. Vielmehr war aufgrund des hohen Dampfdruckes von Cyanwasserstoff (Siedepunkt 26°C) bei einem drucklosen Betrieb mit einem Ausgasen von Cyanwasserstoff im Nachreaktor zu rechnen.

3-Cyano-3,5,5-trimethylcyclohexanon ist ein wichtiges Zwischenprodukt in der Synthese von 5-Amino-1,3,3-trimethyl-cyclohexanmethanamin (Isophorondiamin) und dem entsprechenden Diisocyanat (EP-A-394 967, EP-A-042 119).

### Beispiele

Die angefügte Abbildung verdeutlicht die folgenden Beispiele:

### Beispiel 1

Für die kontinuierliche Synthese wird ein Rührkolben von 80 ml Inhalt (C1) und ein nachgeschalteter Rohrreaktor von 6 m Länge und einem inneren Durchmesser von 4 mm (C2) eingesetzt (s. Fließschema). Beide Reaktoren sind thermostatisch heizbar. Der Rührkolben ist über den mit Sole kühlbaren Kühler W 1 entlüftet.

Aus der gekühlten Vorlage B1 wird über die Pumpe P1 stündlich eine homogene Lösung aus 62,95 g Isophoron und 6,15 g wasserfreiem, mit 50 ppm Phosphorsäure stabilisierten Cyanwasserstoff und über die Mikrodosierpumpe P2 aus der Vorlage B2 0,507 g einer 15 %igen methanolischen Lösung von Natriummethylat in den Rührreaktor C1 zudosiert. Das aus dem Rührreaktor C1 kontinuierlich ablaufende Reaktionsgemisch wird über die Pumpe P3 dem Rohrreaktor C2 zugeführt. Durch separate Heizkreisläufe wird in beiden Reaktoren eine Temperatur von 150°C aufrechterhalten. Der Gehalt an Cyanwasserstoff am Auslauf des Rohrreaktors C2 ist < 50 ppm, das entspricht einem HCN-Umsatz von mehr als 99,9 %.

Das Reaktionsgemisch wird mittels der Mikrodosierpumpe P4 stündlich mit 0,17 g 85 %iger Phosphorsäure versetzt und im Behälter B3 gesammelt.

826 g des stabilisierten Reaktionsgemisches werden fraktioniert destilliert. Nach Abtrennung von 5,1 g Leichtsiedern (Methanol, H₂O) erhält man 383,8 g nicht umgesetztes Isophoron vom Siedepunkt 40 bis 41°C bei 0,5 mbar und 429,4 g 3-Cyano-3,5,5-trimethylcyclohexanon vom Siedepunkt 93 bis 94°C (0,5 mbar). Daraus errechnet sich eine Ausbeute von 96,5 % d. Th. bezogen auf eingesetzten Cyanwasserstoff bzw. 99,4 % d. Th. bezogen auf umgesetztes Isophoron. Der Destillatonsrückstand betägt 7,7 g.

### Beispiel 2

Der in Beispiel 1 beschriebene Rohrreaktor wird auf eine Länge von 3 m verkürzt.

Aus der Vorlage B1 wird über die Pumpe P1 pro Stunde eine Mischung aus 94,43 g Isophoron und 9,23 g Cyanwasserstoff und über die Pumpe P2 aus der Vorlage B2 1,014 g einer 15 %igen Lösung von Natriummethylat in Methanol in den Rührreaktor C1 zudosiert. Das aus dem Reaktor kontinuierlich ablaufende Reaktionsgemisch wird über die Pumpe P3 dem Nachreaktor C2 zugeführt. Beide Reaktoren werden auf einer Reaktionstemperatur von 160°C gehalten. Das in den Sammelbehälter B3 ablaufende, HCN-freie Reaktionsgemisch wird mit 0,34 g/h einer 85 %igen Phosphorsäure stabilisiert.

Nach 20 stündiger Vorlaufzeit werden 840,1 g des stabilisierten Rohproduktes einer fraktionierten Destillation zugeführt. Nach Ab- trennung von 6,9 g Leichtsiedern werden bei einem Vakuum von 1 mbar 385,2 g Isophoron vom Siedepunkt 40 bis 42°C und 437,8 g 3- Cyano-3,5,5-trimethyl-Cyclohexanon vom Siedepunkt 94 bis 95°C abdestilliert, das entspricht einer Ausbeute von 97,1 % d. Th. bezogen auf eingesetzte HCN bzw. 98,9 % d. Th. bezogen auf eingesetztes Isophoron. Der Destillationsrückstand beträgt 10,1 g, das sind 1,2 % bezogen auf eingesetztes Rohprodukt.

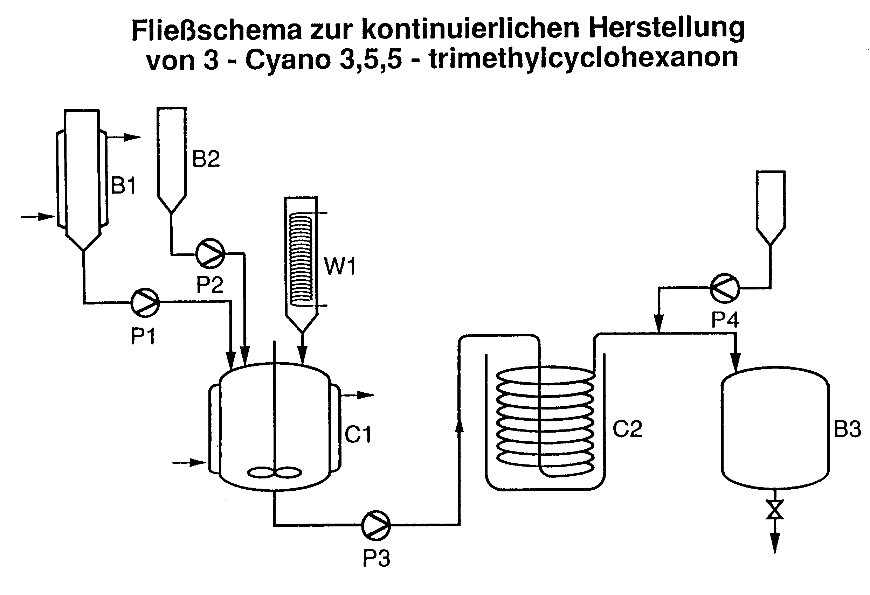

## Patentansprüche

1. Verfahren zur kontinuierlichen Herstellung von 3-Cyano-3,5,5-trimethylcyclohexanon durch basenkatalysierte Umsetzung von Isophoron und Cyanwasserstoff, dadurch gekennzeichnet, daß man in zwei getrennten Reaktionszonen
a) mit im wesentlichen vollständiger Rückvermischung und anschließend
b) im wesentlichen ohne Rückvermischung
arbeitet.

2. Verfahren nach Anspruch 1, dadurch gekennzeichnet, daß man als Reaktionszone b) einen zylindrischen Reaktor mit Füllkörpern verwendet.

3. Verfahren nach Anspruch 1, dadurch gekennzeichnet, daß man als Reaktionszone b) einen zylindrischen Reaktor mit festen Einbauten verwendet.

## Claims

1. A process for the continuous preparation of 3-cyano-3,5,5-trimethylcyclohexanone by base-catalyzed reaction of isophorone and hydrogen cyanide, which comprises carrying out the reaction in two separate reaction zones
a) with essentially complete back-mixing and subsequently
b) essentially without back-mixing.

2. A process as claimed in claim 1, wherein the reaction zone b) is a cylindrical reactor containing packing elements.

3. A process as claimed in claim 1, wherein the reaction zone b) is a cylindrical reactor containing fixed internals.

## Revendications

1. Procédé de préparation continue de 3-cyano-3,5,5-triméthylcyclohexanone par réaction d'isophorone et d'acide cyanhydrique catalysée par une base, caractérisé en ce que l'on procède dans deux zones de réaction séparées
a) avec remélangeage pratiquement complet, puis
b) pratiquement sans remélangeage.

2. Procédé selon la revendication 1, caractérisé en ce que l'on utilise, comme zone de réaction b), un réacteur cylindrique contenant des corps de garnissage.

3. Procédé selon la revendication 1, caractérisé en ce que l'on utilise, comme zone de réaction b), un réacteur cylindrique contenant des inserts fixes.
